# EUROPÄISCHE PATENTSCHRIFT

(11) **EP 0 998 270 B1**
(45) Veröffentlichungstag und Bekanntmachung des Hinweises auf die Patenterteilung: **12.01.2005**
(21) Anmeldenummer: 98929190.1
(22) Anmeldetag: 06.07.1998
(51) Int. Cl.: A61K 9/20, A61K 9/48

(54) **VERFAHREN UND VORRICHTUNG ZUM HERSTELLEN EINER MEHRSCHICHTIGEN, PHYSIOLOGISCH VERTRÄGLICHEN DARREICHUNGSFORM**
METHOD AND DEVICE FOR PRODUCING A MULTI-LAYER, PHYSIOLOGICALLY TOLERATED PRESENTATION FORM
PROCEDE ET DISPOSITIF POUR PRODUIRE UNE FORME GALENIQUE MULTICOUCHE, PHYSIOLOGIQUEMENT TOLERABLE

(30) Priorität: 09.07.1997 EP 97111668
(43) Veröffentlichungstag der Anmeldung: 10.05.2000
(73) Patentinhaber: Swiss Caps Rechte und Lizenzen AG, 9533 Kirchberg (CH)
(72) Erfinder: GREITHER, Peter, CH-9533 Kirchberg (CH); ENGEL, Dieter, Wolfgang, CH-9524 Zuzwil (CH); BROCKER, Erich, CH-9533 Kirchberg (CH); TOMKA, Ivan, CH-8702 Zollikon (CH); MENARD, Rico, CH-8044 Zürich (CH)
(74) Vertreter: Wenger, René
(86) Internationale Anmeldenummer: PCT/CH1998/000294
(87) Internationale Veröffentlichungsnummer: WO 1999/002136

(56) Entgegenhaltungen:
- EP-A- 0 198 493
- EP-A- 0 755 763
- WO-A-89/12442
- WO-A-93/13761
- WO-A-96/40083
- WO-A-97/15293
- FR-A- 2 125 300
- GB-A- 2 178 996
- US-A- 4 352 823
- NETSTAL NEWS, Bd. 31, April 1997, XP002081709 www.netstal.com

## Beschreibung

Die Erfindung betrifft ein Verfahren zum Herstellen einer mehrschichtigen, physiologisch verträglichen Darreichungsform gemäss dem Oberbegriff von Anspruch 1. Derartige Darreichungsformen werden vor allem zur Verabreichung von Arzneimitteln eingesetzt. Sie können aber auch dazu dienen, diätetischen Nährmittel oder auch nur reine Genussmittel aufzunehmen. Die aktive Komponente ist dabei in der Regel in der Kernkomponente enthalten, wobei die Hüllkomponente primär eine Schutzfunktion aufweist. Bei Arzneimitteln wird die Hüllkomponente auch zum Erzielen einer Retardwirkung eingesetzt.

Darreichungsformen der genannten Art treten vor allem als Kapseln aus Stärke, Gelatine oder dergleichen in Erscheinung. Es sind bereits zahlreiche Kapselherstellungsverfahren bekannt, wie z.B. das Rotary-Die-Verfahren für Weichgelatinekapseln oder das Tauchverfahren für Hartgelatinekapseln. Bei Hartgelatinekapseln ist es auch bereits bekannt, die harte Aussenhülle der Kapsel in einem Spritzgiessverfahren herzustellen (EP-A-118 240). Alle bisher bekannten Verfahren haben jedoch den Nachteil, dass es sich dabei um Prozesse handelt, die lediglich in mehreren, teilweise aufwendigen Schritten zur endgültigen Darreichungsform führen. Teilweise entstehen dabei erhebliche Materialabfälle wie z.B. beim Rotary-Die-Verfahren und im übrigen ist der Einsatz der verwendeten Stoffe, bedingt durch die jeweils eingesetzten Verfahren, stark eingeschränkt.

Die GB-A-2 207 355 beschreibt die Herstellung einer ringförmigen Darreichungsform zur kontrollierten Abgabe einer therapeutisch wirksamen Substanz im menschlichen oder tierischen Körper. Der Ring wird aus einem koextrudierten Materialstrang bestehend aus einem Kernmaterial und einem Hüllmaterial gebildet, wobei die beiden Enden des Materialstranges miteinander verbunden werden. Ersichtlicherweise sind dabei mehrere Arbeitsschritte erforderlich, nämlich Koextrudieren eines endlosen Materialstrangs, Ablängen eines Teilstrangs und Zusammensetzen des Teilstrangs.

Die WO 89/12 442 betrifft eine Darreichungsform für die Behandlung von Fischen. Sie besteht aus einem koextrudierten Hohlkörper aus einem tierischen oder pflanzlichen Material und aus einem vollständig darin eingeschlossenen Wirkstoff. Der Hohlkörper enthält aber auch noch einen Lufteinschluss, damit das Gebilde an der Wasseroberfläche schwimmt und von den Fischen besser angenommen wird. Um die gewünschte Wasserdichtigkeit zu erreichen, muss der koextrudierte Materialstrang zu geschlossenen Kammern in einem weiteren Vorgang umgeformt werden.

Die WO 97/15 293 offenbart ein Verfahren zur Herstellung von mehrschichtigen, festen Arzneiformen zur oralen oder rektalen Verabreichung. Das Erzeugnis wird zuerst aus einem gemeinsamen Koextrusionswerkzeug zweischichtig extrudiert und dann mit Hilfe von Quetschbalken oder Quetschwalzen zu kapselähnlichen Gebilden abgequetscht. Dabei entstehen ersichtlicher- weise Quetschnähte ähnlich wie beim Rotary-Die-Verfahren.

Durch die WO 93/13761 ist ein Verfahren zum Herstellen von Weichgelatinekapseln nach einem Tropfverfahren bekannt geworden. Dabei wird das vorzugsweise pastöse oder flüssige Füllgut mit einer Weichgelatinemasse umhüllt, welche anschliessend in einem Kühlbad verfestigt wird.

Die WO 96/40083 beschreibt eine Gelatinekapsel, bei welcher eine Sperrschicht verhindert, dass hydrophile oder hydrophobe Komponenten der Kapselfüllung in die Aussenschicht eindringen können.

Die US-A-4,352,823 betrifft einen koextrudierten Kaugummi bestehend aus einer relativ weichen Kernmasse und einer trockenen Aussenhülle aus härterem Material. Abgesehen davon, dass das Koextrudat nachträglich zu verpackbaren Körpern geformt werden muss, lässt sich diese technische Lehre nicht auf die Herstellung einer physiologisch verträglichen Darreichungsform übertragen.

Schliesslich ist durch die US-A-5 650 232 ein Verfahren zum Herstellen von nahtlosen, zweiphasigen Kapseln bekannt geworden. Dabei werden ebenfalls zwei Materialstränge in einer gemeinsamen Düse koextrudiert. Das Extrudat fällt im freien Fall aus der Extrusionsdüse, wobei sich infolge der Grenzflächenspannung Körper bilden, bei denen die Aussenhülle das Kernmaterial völlig umgibt. Diesem Verfahren sind im Hinblick auf die dabei zu berücksichtigenden Materialeigenschaften sehr enge Grenzen gesetzt. Ausserdem besteht keine Kontrolle über die endgültige Aussenkontur der Kapseln.

Es ist daher eine Aufgabe der Erfindung, ein Verfahren der eingangs genannten Art zu schaffen, das den Arbeitsprozess wesentlich vereinfacht und mit dessen Hilfe ein breiteres Spektrum von Stoffen verarbeitbar ist. Ausserdem sollen dabei nahtlose Körper mit möglichst homogenen Phasen erzeugt werden. Diese Aufgabe wird mit einem Verfahren gelöst, das die Merkmale im Anspruch 1 aufweist.

Das Einspritzen der Kernkomponente und der Hüllkomponente in eine gemeinsame Werkzeugkavität hat den Vorteil, dass es sich um einen Arbeitsprozess handelt, der im Gegensatz zu den erwähnten Koextrusionsverfahren unmittelbar zum fertigen Erzeugnis führt. Die thermoplastische Aufbereitung wenigstens der Hüllkomponente lässt sich vergleichsweise mit einfachen Mitteln realisieren. Mit dem hier vorgestellten Verfahren können Kapseln praktisch in einem einzigen Arbeitsgang hergestellt und gefüllt werden, wobei die harte Aussenhülle keine Naht aufweist wie bei den bekannten Steckkapseln. Die Aussenform der Kapsel ist bezüglich Durchmesser und Länge innerhalb einer grossen Bandbreite beliebig wählbar und es können auch Materialien eingespritzt werden, die sich beispielsweise im Rotary-die-Verfahren nicht verarbeiten lassen. Dadurch kann die immer noch weit verbreitete Gelatine durch ein preiswerteres und einfacher herzustellendes Biopolymer ersetzt werden.

Die Aufbereitung und Verarbeitung von Biopolymeren zu deren Überführung in einen spritzbaren Zustand sind in der EP-A-118 240 und WO 90/14938 am Beispiel von Stärken beschrieben. Diesbezüglich und insbesondere für die Definitionen der Ausdrücke "Schmelzströme" und "plastisch" bzw. "thermoplastisch" soll ausdrücklich auf diese beiden Dokumente verwiesen werden.

Vorzugsweise wird die Kernkomponente in einer ersten Zuführvorrichtung und die Hüllkomponente im thermoplastischen Zustand in einer zweiten Zuführvorrichtung bereitgestellt. Anschliessend werden die beiden Komponenten simultan oder sequentiell derart in die Werkzeugkavität eingespritzt, dass spätestens dort die Kernkomponente vollständig von der Hüllkomponente umgeben wird. Zuletzt wird der eingespritzte Formling abgekühlt und nach dem Öffnen der Werkzeugkavität ausgeworfen. Selbstverständlich kann die Kernkomponente dabei von mehreren Hüllkomponenten umgeben sein. Auch die Kernkomponente selbst könnte in sich in mehrere Phasen aufgeteilt sein.

Die Kernkomponente und die Hüllkomponente können dabei als separate Schmelzströme über einen gemeinsamen Einspritzkopf in die Werkzeugkavität eingespritzt werden. Die vollständige Umhüllung der Kernkomponente findet dabei in der Werkzeugkavität statt. Es ist aber auch denkbar, dass die beiden Komponenten als gemeinsamer Schmelzstrom über einen gemeinsamen Einspritzkopf in die Werkzeugkavität eingespritzt werden. Zu diesem Zweck muss der Schmelzstrom der Hüllkomponente am gemeinsamen Einspritzkopf in regelmässigen Abständen mit Material der Kernkomponente geimpft werden.

Die Kernkomponente wird dabei bereits im Einspritzkopf von der Hüllkomponente umgeben, wobei der gemeinsame Schmelzstrom in die geöffnete Werkzeugkavität eingespritzt wird und die Aussenform durch Schliessen der Schieber erzielt wird.

Alternativ kann die Werkzeugkavität aber auch über eine Einspritzöffnung zuerst vollständig mit der Hüllkomponente gefüllt werden. Sodann wird die Kernkomponente über eine Einspritznadel in die Hüllkomponente bzw. in die Werkzeugkavität eingespritzt, wobei die dabei verdrängte Hüllkomponente über die Einspritzöffnung aus der Werkzeugkavität abfliesst. Dabei wäre es denkbar, auch sehr dünnflüssige Substanzen zu verkapseln, die nicht thermoplastisch aufbereitbar sind.

Vorzugsweise wird wenigstens die Hüllkomponente in einem Extruder thermoplastisch aufbereitet, wobei der Einspritzdruck am Extruder erzeugt wird. Derartige Extruder mit Schubschnecken sind beispielsweise aus der Spritzgusstechnik für Kunststoffmaterialien bereits bekannt. Dagegen werden dünnflüssige Kernkomponenten vorteilhaft mittels einer Dosierkolbenpumpe zudosiert. Dabei lassen sich die einzuspritzenden Mengen sehr exakt festlegen.

Die Erfindung betrifft auch eine Vorrichtung zum Herstellen einer mehrschichtigen, physiologisch verträglichen Darreichungsform, welche durch die Merkmale in Anspruch 9 gekennzeichnet ist. Die dabei eingesetzten Werkzeuge sind je nach dem gewählten Verfahren unterschiedlich ausgestattet. Es kann sich um ein Heisskanaleinspritzwerkzeug handeln, wie es in ähnlicher Form schon zum Spritzen von Kunststoffartikeln bekannt ist. Besonderes Augenmerk ist dabei auf die Ausstossvorrichtung zu richten, damit die relativ empfindlichen Formkörper beim Ausstossen nicht beschädigt werden.

Alternativ kann das Werkzeug aber auch mit speziellen Einspritzköpfen, Schiebern und Einspritznadeln versehen sein.

In der Spritzgusstechnik sind Vorrichtungen für das Herstellen mehrschichtiger Körper aus unterschiedlichen Werkstoffen im sogenannten Co-Injection-Verfahren schon seit langem bekannt. So zeigt beispielsweise die EP-A-755763 ein Werkzeug bei dem der Formraum nach dem Einspritzen der Kernkomponente und der Hüllkomponente durch Zusammenschieben von Werkzeugteilen verkleinert werden kann, wobei der Formling die endgültige Aussenkontur erhält.

Die EP-A-198 493 betrifft einen luftschalldämmenden und körperschalldämmenden Formling, insbesondere für Kraftfahrzeugteile, der mehrschichtig aufgebaut ist. Die mittlere Zone aus elastischem Material und die Aussenzone kann gleichzeitig durch Einspritzen von Polymerschmelzen mittels einer Zweikanalspritzdüse innerhalb eines Spritzgiesswerkzeuges geformt werden.

Die FR-A-2.125.300 zeigt ein Spritzgiesswerkzeug, mit dessen Hilfe beispielsweise Schuhabsätze aus unterschiedlichen Materialien hergestellt werden können. Eine innere Komponente kann dabei mittels einer Einspritznadel in den Formhohlraum eingespritzt werden.

Die mit dem beschriebenen Verfahren erhältliche Darreichungsform kann so beschaffen sein, dass sie oral, rektal oder vaginal vom menschlichen oder tierischen Körper aufgenommen werden kann. Wie bei konventionellen Darreichungsformen kann die äussere Erscheinung, der Geschmack oder die Oberflächenbeschaffenheit durch entsprechende Hilfsstoffe beeinflusst werden. Die Kernkomponente und die Hüllkomponente sind vorzugsweise in physiologischer Umgebung vollständig absorbierbar.

Die Kernkomponente kann in pastöser oder flüssiger Form oder als Kolloidsystem vorliegen. Denkbar sind dabei insbesondere Dispersionen, Suspensionen, Emulsionen und feste oder flüssige Schäume. Ausserdem enthält vorzugsweise die Kernkomponente wenigstens einen pharmazeutischen Wirkstoff oder eine andere biologisch wirksame Substanz. Es ist aber auch denkbar, dass die Hüllkomponente ebenfalls einen Wirkstoff aufweist der gegebenenfalls zusammen mit dem Wirkstoff der Kernkomponente in eine Wechselwirkung tritt.

Weitere Vorteile ergeben sich, wenn die Hüllkomponente ein Retardmittel für die verzögerte Wirkstofffreisetzung im Magen-Darmtrakt in gelöster Form enthält. Dabei fällt das bisher übliche nachträgliche Beschichten der fertigen Kapseln ganz weg.

Die Hüllkomponente besteht grundsätzlich aus physiologisch verträglichen Stoffen, bevorzugt aus einem synthetischen Polymer oder Biopolymer, insbesondere
- Polysaccharide aus der Gruppe der Polyglucosane wie z.B. native Stärken und native Cellulose; Polysaccharide aus der Gruppe der Polygalactomannane wie z.B. nativer Guaroder Tara-gum, Polysaccharide aus der Gruppe der Glucomannane, weiterhin Polysaccharide wie Pectine, Alginsäure, Alginate, Chitin, Chitosan, Gummi-arabicum
- Oligosaccharide aus der Gruppe der modifizierten bereits genannten Polysaccharide insbesondere thermisch, enzymatisch, säurehydrolytisch, oxidativ oder mechanisch depolymerisierte Stärken oder Cellulose, Quellstärken usw.
- Oligo- und Polysaccaridderivate wie z.B. Ester, Ether und Acetate der bereits genannten Substanzen
- Proteine wie Albumin, Casein, Glutin, Zeins
- Proteinderivate wie Amide, Ester und Schiff' sehe Basen
- natürliche Ester wie Schellack
- Polyester wie Succinsäure-, Milchsäure-, Buttersäure-, Fettsäureester von Alkanolen, Polylactome
- Polyterpene, wie Polyisoprene
in Kombination mit Weichmachern wie Wasser, Alkohol, Säuren, Estern und Ethern.

Je nach Anwendungszweck lassen sich diese Stoffe relativ leicht thermoplastisch aufbereiten bzw. verarbeiten.

Ausführungsbeispiele der Erfindung sind in den Zeichnung dargestellt und werden nachstehend genauer beschrieben. Es zeigen:
- Figur 1: Die schematische Darstellung eines Spritzgusswerkzeugs mit zwei Extrudern,
- Figur 2: ein Querschnitt durch eine typische Darreichungsform,
- Figur 3: ein Querschnitt durch ein alternatives Spritzgusswerkzeug in einer ersten Betriebsphase,
- Figur 4: das Spritzgusswerkzeug gemäss Figur 3 in einer zweiten Betriebsphase, und
- Figur 5: ein weiteres Ausführungsbeispiel eines Spritzgusswerkzeuges zum separaten Einspritzen der Kernkomponente.

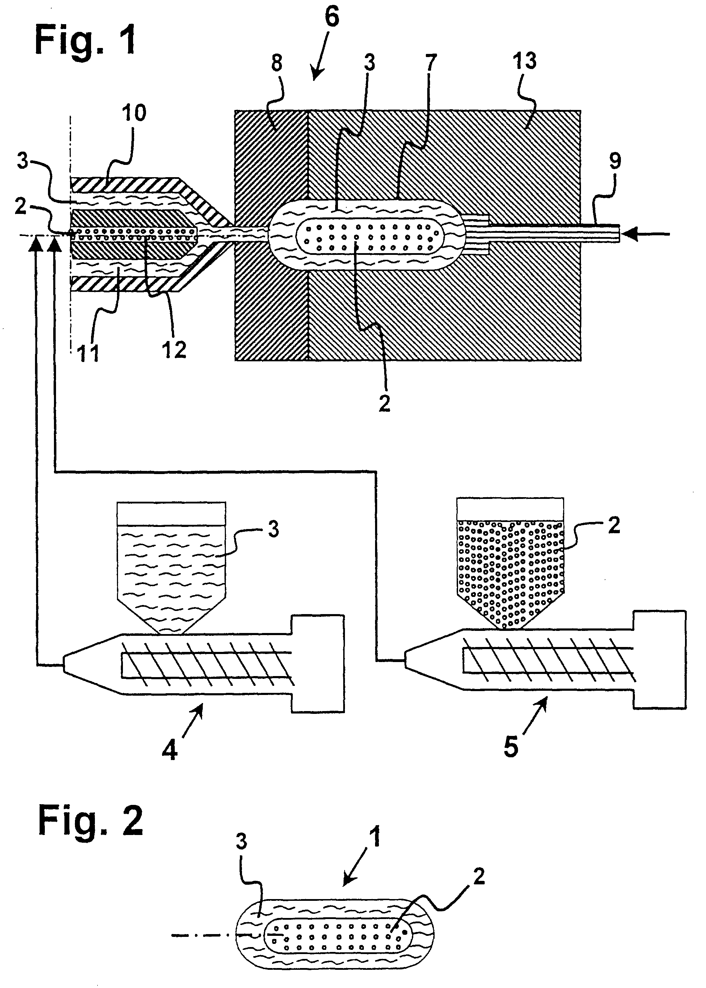

Figur 1 zeigt stark vereinfacht ein Spritzgusswerkeug 6 bestehen aus einem Werkzeugblock 13 und einer Einspritzplatte 8, die zusammen eine Werkzeugkavität 7 bilden. Einspritzplatte und Werkzeugblock können durch hier nicht näher dargestellte Mittel zum Öffnen der Werkzeugkavität 7 auseinandergefahren werden. Das Ausstossen des fertigen Formlings erfolgt über einen Ausstosser 9.

An der Einspritzplatte 8 liegt ein Düsenkopf 10 an, der über einen äusseren Einspritzkanal 11 und über einen inneren Einspritzkanal 12 verfügt. Die Einspritzkanäle sind je mit einem ersten Extruder 4 für die Hüllkomponente 3 und mit einem zweiten Extruder 5 für die Kernkomponente 2 verbunden.

Die beiden Komponenten werden in den Extrudern thermoplastisch aufbereitet und über den Düsenkopf 10 in separaten Schmelzströmen sequentiell in die Werkzeugkavität 7 eingespritzt. Dabei wird vorzugsweise zuerst eine dosierte Menge der Hüllkomponente 3 und anschliessend eine dosierte Menge Kernkomponente 2 unter einem bestimmten Einspritzdruck eingespritzt. Der Einspritzkopf ist stark vereinfacht dargestellt. In Wirklichkeit haben die relativ zueinander verschiebbaren Bauteile des Kopfs eine Ventilfunktion, wobei im Zentrum auch noch eine Ventilnadel vorgesehen ist. Ein derartiger Mehrkanal-Einspritzkopf ist beispielsweise in der EP-A-647 514 beschrieben. Selbstverständlich können je nach Konstruktion mehrere Hüllkomponenten übereinander gespritzt werden. Durch die richtige Wahl der relativen Viskosität, der Temperatur und der Fliessgeschwindigkeit der beiden Komponenten, sowie durch eine sehr genaue Abstimmung der Dosiervolumina, wird einerseits eine vollständige Umhüllung der Kernkomponente in der Werkzeugkavität erreicht und andererseits eine Vermischung der beiden Komponenten vor dem Erstarren verhindert. Es hat sich auch als besonders vorteilhaft erwiesen, nach dem Einspritzen der Kernkomponente in die Hüllkomponente nochmals eine geringe Menge Hüllkomponente nachzuspritzen, um auch im Bereich der Einspritzöffnung eine vollständige Umhüllung zu gewährleisten. Nach dem Abkühlen des Formlings wird das Werkzeug geöffnet und die in Figur 2 dargestellte Kapsel 1 mit Hilfe des Ausstossers 9 aus der Werkzeugkavität ausgeworfen. Selbstverständlich ist dabei jedes Werkzeug mit mehreren Kavitäten versehen und die Öffnungs- und Schliessvorgänge erfolgen automatisch, so dass eine rationelle Arbeitsweise möglich ist.

Die Trennebene der Werkzeuge wird so gewählt, dass der Formling beim Öffnen nicht herausgezogen wird, sondern im Werkzeugblock 13 verbleibt. Selbstverständlich ist dabei auch die Aussenkontur zu berücksichtigen, wobei alle gängigen Kapselformen wie Oblongs, Kugeln, Ampullen oder auch Sonderformen spritzbar sind.

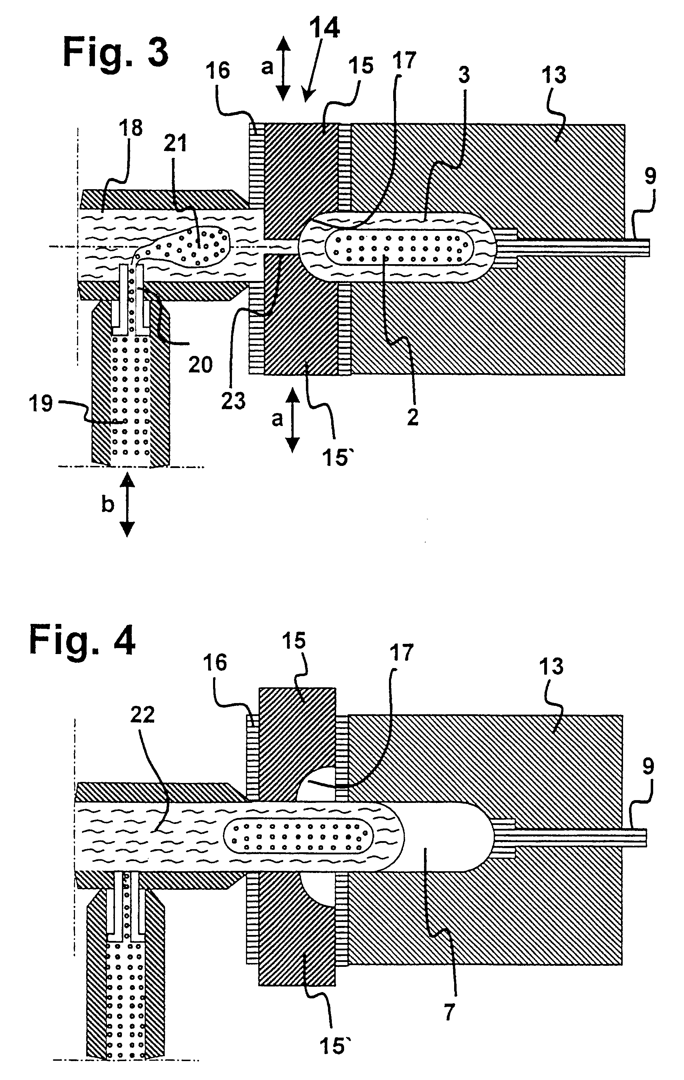

Beim Ausführungsbeispiel gemäss den Figuren 3 und 4 werden die beiden Komponenten in einem gemeinsamen Schmelzstrom in die Werkzeugkavität eingespritzt. An Stelle der normalen Einspritzplatte tritt eine Schieberplatte 14 mit wenigstens zwei Schiebern 15, 15', die in Pfeilrichtung a in einer Schieberführung 16 verschiebbar sind. Die geschlossenen Schieber bilden ebenfalls einen Teil der Begrenzungsfläche 17 der Werkzeugkavität, sowie eine Rückflussöffnung 23. Im geöffneten Zustand gemäss Figur 4 bilden die Schieber 15, 15' eine Einspritzöffnung, deren Querschnitt etwa dem grössten Querschnitt der Werkzeugkavität entspricht.

An der Schieberplatte 14 liegt ein Haupteinspritzkanal 18 an, der mit einem hier nicht mehr dargestellten Extruder für die Hüllkomponente 3 verbunden ist. Seitlich ist der Haupteinspritzkanal 18 mit einem Seiteneinspritzkanal 19 versehen, der mit einem weiteren Extruder für die Kernkomponente 2 verbunden ist. Der Seiteneinspritzkanal verfügt über eine in Pfeilrichtung b verschiebbare Nadel 20, deren Spitze in den Haupteinspritzkanal 18 eingeführt werden kann.

Im Betriebszustand gemäss Figur 3 sind die Schieber 15, 15' bis zum Erkalten des bereits in die Kavität eingespritzten Formlings geschlossen. Gleichzeitig wird bereits am Haupteinspritzkanal 18 mit vorgeschobener Einspritznadel 20 eine bestimmte Menge der Kernkomponente 2 in die Hüllenkomponente 3 eingespritzt. Dabei bildet sich eine Blase 21, die vollständig von der Hüllkomponente umgeben ist.

Nach dem Auswerfen des fertigen Formlings aus der Werkzeugkavität werden die beiden Schieber 15, 15' geöffnet und die beiden Komponenten werden als gemeinsamer Schmelzstrom in die werkzeugkavität eingepresst. Aufgrund der so entstehenden Eintrittsöffnung muss der gemeinsame Schmelzstrom dabei keine Engstelle passieren, so dass die Blase 21 ihre Form beibehält. Zum Einspritzen des Schmelzstroms wird die Nadel 20 aus dem Haupteinspritzkanal 18 zurückgezogen.

Die endgültige Form der Kapsel wird anschliessend durch das Zusammenführen der beiden Schieber 15, 15' definiert, wobei verdrängtes Material der Hüllkomponente 3 durch die Rückflussöffnung 23 zurückströmen kann. Über den Seiteneinspritzkanal 19 könnte auch eine sehr dünnflüssige Komponente eingespritzt werden, die sich nicht in einem Extruder aufbereiten lässt. In einem derartigen Fall tritt anstelle des Extruders vorteilhaft eine Dosierkolbenpumpe.

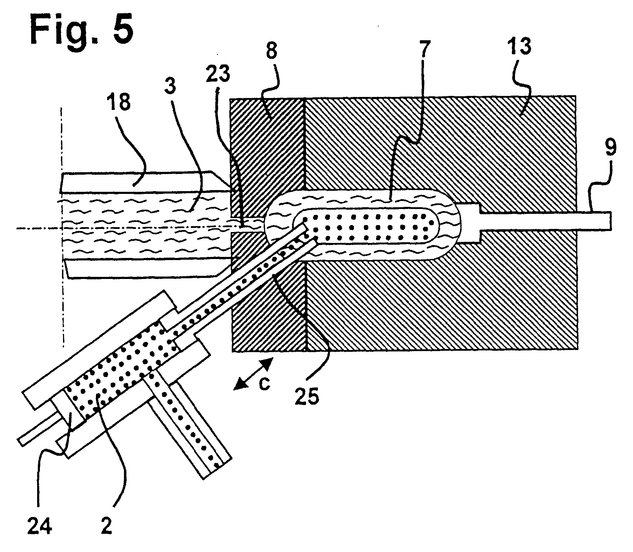

Beim Ausführungsbeispiel gemäss Figur 5 ist eine normale Einspritzplatte vorgesehen, die mit einem Haupteinspritzkanal 18 verbunden ist. Über diesen Kanal wird die Hüllkomponente in die Kavität eingespritzt. Die Einspritzung der Kernkomponente erfolgt seitlich mit einer Nadel 25 direkt in die Kavität bzw. in die Hüllkomponente. Die Nadel 25 ist zu diesem Zweck ebenfalls in Pfeilrichtung c verschiebbar. Die Kernkomponente 2 wird mittels einer Dosierkolbenpumpe 24 zudosiert.

Im Betrieb wird bei geschlossenem Werkzeug zunächst die Werkzeugkavität 7 über den Haupteinspritzkanal 18 vollständig mit der Hüllkomponente 3 gefüllt. In einem nächsten Schritt wird über die vorgeschobene Einspritznadel 25 die Kernkomponente eingespritzt, wobei ein Teil der Hüllkomponente verdrängt wird und über die Rückflussöffnung 23 zurückfliesst. Zum Abkühlen des Formlings wird die Nadel 25 zurückgezogen. Ersichtlicherweise muss der Einspritzdruck für die Kernkomponente 2 grösser sein als der am Hauptkanal 18 anliegende Druck. Die Erfindung wird nachstehend anhand von verschiedenen Ausführungsbeispielen weiter dokumentiert.

### Beispiele:

### Beispiel 1

6,5 kg/h native Kartoffelstärke (st) mit einem Wassergehalt von 6 % werden mit 3,5 kg/h Glycerol (gly) 98 bis 101 % (vgl. Deutsches Arzneimittelbuch) der ersten der beiden Extruder der Gesamtanlage zugeführt. Der zweite Extruder wird mit 20 kg/h Polyethylenglycol (PEG) 6000 (Macrogol 6000 DAB) UNITED STATES PHARMACOPIA (USP) mit 0,2 kg/h Hydrocortisonacetat (USP) beschickt. Die Schmelze des ersten Extruders wird bei 150°C und die des zweiten Extruders bei 65°C gespritzt. Der Einspritzdruck beträgt in beiden Extrudern 50 bar. 3000 Darreichungsformen pro Stunde werden mit einem 16 Kavitäten enthaltenen Spritzgusswerkzeug erhalten.

Zur Durchführung von in Vitro-Untersuchungen werden die oval geformten Darreichungsformen in 0,1 N Salzsäure bei 36°C gelöst. 90 % des Materials der Darreichungsform sind in 30 Minuten gelöst (Lösedauer).

In den nachfolgenden Beispielen wurden die oben genannten Inhaltsstoffe und Parameter variiert:

### Beispiel 2 bis 4:

| Beispiel | Extruder 1 Komponenten T [°C] | Extruder 2 Komponenten T [°C] | Lösedauer für > 90% |
|---|---|---|---|
| 2 | 6kg st + 3 kg gly 160 | 18kg PEG + 0.05 kg von (1) 40 | 60 min |
| 3 | 5kg von (2) + 2kg von (3) 130 | 20kg von (4) + 2kg von (5) 100 | 60 min |
| 4 | 6kg von (6) + 1kg gly 180 | 10kg von (7) + 3kg von (8) 100 | 60 min |
| (1) Nifedipin nicht mikronisiert (USP); (2) Guar Gum; (3) 25 Gewichtsteile von Mono-, Di-, Triethyl Estern der Zitronensäure + 1 Gewichtsteil Pfefferminzöl + 4 Gewichtsteile Pfefferminzaroma; (4) Acetaminophen (USP); (5) 1 Gewichtsteil N-Methyl-2-Pyrrolidon (Pharmasolve USP) + 1 Gewichtsteil Crospovidon (USP); (6) Gelatine; (7) Silikonöl; (8) Nystatin. | | | |

### Beispiele 5 - 6:

In den folgenden Beispielen wurden zwei Schutzhüllen appliziert und die Wirksubstanz wurde dem dritten Extruder beigegeben:

| Bsp. | Extruder 1 Komponenten T [°C] | Extruder 2 Komponenten T [°C] | Extruder 3 Komponenten T [°C] |
|---|---|---|---|
| 5 | 6kg st + 3 kg gly 150 | 0.5kg von (3) + 0.5kg von (4) 60 | 15kg von (1) + 10kg von (2) 15 |
| 6 | 5kg von (5) + 1kg von (6) 130 | 3kg von (4) -10 | 20kg von (7) + 5kg von (8) 15 |
| (1) 1 Gewichtsteil Cyclosporin A; (2) 1 Gewichtsteil Ethanol 96 % Ph.Eur. + 1 Gewichtsteil Propylenglycol (USP) + 10 Gewichtsteile Solutol Ph.Eur.; (3) Bienenwachs; (4) hydriertes Soyabohnenöl; (5) Hydroxypropylmethylzellulose; (6) 5 Gewichtsteile Propylenglycol (USP) + 10 Gewichtsteile Siliciumdioxid (USP) + 2 Gewichtsteile Eisenoxid NF + 2 Gewichtsteile Titaniumdioxide (USP); (7) 30 Gewichtsteile Kalziumascorbat + 1.7 Gewichtsteile Thiamin Hydrochlorid + 2 Gewichtsteile Riboflavin + 1.7 Gewichtsteile Pyridoxin Hydrochlorid + 15 Gewichtsteile Niacin + 8 Gewichtsteile Kalzium Pantothenate + 0.2 Gewichtsteile Biotin + 0.4 Gewichtsteile Folsäure + 15 Gewichtsteile DL-α-Tocopherolacetat + 15 Gewichtsteile β-Karotin 30 % susp. + 60 Gewichtsteile Magnesiumcarbonat + 60 Gewichtsteile Kalziumcarbonat + 10 Gewichtsteile Eisenfumarat; (8) 131 Gewichtsteile Soyabohnenöl + 35 Gewichtsteile hydriertes Soyabohnenöl + 7,5 Gewichtsteile Lecitin der Soyabohne + 7.5 Gewichtsteile Bienenwachs. | | | |

Im Vergleich zur direkten Einnahme der Wirksubstanzen (vgl. 1 bis 8) konnte durch die Darreichungsform mit zwei Hüllschichten eine verzögerte Freisetzung und Lösung der Wirksubstanzen (Retardwirkung) erreicht werden.

## Patentansprüche

1. Verfahren zum Herstellen einer mehrschichtigen, physiologisch verträglichen Darreichungsform, insbesondere einer Kapsel, bei dem wenigstens eine Kernkomponente von wenigstens einer Hüllkomponente vollständig umhüllt wird, **gekennzeichnet durch** Einspritzen der Kernkomponente (2) und der Hüllkomponente (3) in eine gemeinsame Werkzeugkavität (7), wobei wenigstens die Hüllkomponente thermoplastisch aufbereitet ist.

2. Verfahren nach Anspruch 1, **dadurch gekennzeichnet,**
- **dass** die Kernkomponente in einer ersten Zuführvorrichtung (4) bereitgestellt wird,
- **dass** die Hüllkomponente in thermoplastischem Zustand in einer zweiten Zuführvorrichtung (5) bereitgestellt wird,
- **dass** die Kernkomponente und die Hüllkomponente simultan oder sequentiell derart in die Werkzeugkavität eingespritzt werden, dass spätestens dort die Kernkomponente vollständig von der Hüllkomponente umgeben wird,
- und das der eingespritzte Formling (1) abgekühlt und nach dem Öffnen der Werkzeugkavität (7) ausgeworfen wird.

3. Verfahren nach Anspruch 2, **dadurch gekennzeichnet, dass** die Kernkomponente und die Hüllkomponente als separate Schmelzströme über einen gemeinsamen Einspritzkopf (10) in die Werkzeugkavität (7) eingespritzt werden.

4. Verfahren nach Anspruch 2, **dadurch gekennzeichnet, dass** die Kernkomponente und die Hüllkomponente als gemeinsamer Schmelzstrom über einen gemeinsamen Einspritzkopf in die Werkzeugkavität eingespritzt werden.

5. Verfahren nach Anspruch 4, **dadurch gekennzeichnet, dass** die Kernkomponente bereits im Einspritzkopf von der Hüllkomponente umgeben wird, wobei der gemeinsame Schmelzstrom in die geöffnete Werkzeugkavität eingespritzt wird.

6. Verfahren nach Anspruch 2, **dadurch gekennzeichnet, dass** die Werkzeugkavität über eine Einspritzöffnung zuerst vollständig mit der Hüllkomponente gefüllt wird und dass sodann die Kernkomponente über eine Einspritznadel in die Hüllkomponente eingespritzt wird, wobei die dabei verdrängte Hüllkomponente über die Einspritzöffnung aus der Werkzeugkavität abfliesst.

7. Verfahren nach einem der Ansprüche 1 bis 6, **dadurch gekennzeichnet, dass** wenigstens die Hüllkomponente in einem Extruder thermoplastisch aufbereitet wird und dass der Einspritzdruck am Extruder erzeugt wird.

8. Verfahren nach einem der Ansprüche 1 bis 7, **dadurch gekennzeichnet, dass** die Kernkomponente mittels einer Dosierkolbenpumpe zudosiert wird.

9. Vorrichtung zum Herstellen einer mehrschichtigen, physiologisch verträglichen Darreichungsform, nämlich einer Kapsel, mit wenigstens einer Kernkomponente, die von wenigstens einer Hüllkomponente vollständig umhüllt ist, zur Durchführung des Verfahrens nach einem der Ansprüche 1 bis 8, **gekennzeichnet durch**
- eine erste Zuführvorrichtung (4)in der Form eines Extruders für die Hüllkomponente,
- eine zweite Zuführvorrichtung in der Form eines Extruders (5) oder einer Dosierkolbenpumpe (24) für die Kernkomponente,
- ein Werkzeug (6) bestehend aus einem Werkzeugblock (13) und einer Einspritzplatte (8) oder einer Schieberplatte (14) die zusammen wenigstens eine gemeinsame Werkzeugkavität (7) zur Aufnahme beider Komponenten bilden,
- eine Werkzeugbetätigung zum Öffnen und Schliessen des Werkzeuges, und
- ein im Werkzeugblock gelagerter Ausstosser (9) zum Auswerfen der fertigen Kapsel aus der Werkzeugkavität, wobei die Werkzeugkavität 87) die Form einer runden, ovalen, oblongen oder ampullenförmigen Kapsel aufweist, und wobei die Trennebene zwischen Werkzeugblock und Einspritzplatte oder Schieberplatte so gewählt wird, dass sie **durch** die Werkzeugkavität verläuft und dass die Kapsel beim Öffnen des Werkzeugs im Werkzeugblock verbleibt und **durch** den Ausstosser gegen die Trennebene hin ausstossbar ist.

10. Vorrichtung nach Anspruch 9, **dadurch gekennzeichnet, dass** die Kernkomponente mit der Dosierkolbenpumpe entweder in die Werkzeugkavität oder in den Zufuhrkanal der ersten Zuführvorrichtung zudosierbar ist.

11. Vorrichtung nach Anspruch 9, **dadurch gekennzeichnet, dass** das Werkzeug eine Schieberplatte mit wenigstens zwei Schiebern (15, 15') aufweist, welche in geschlossenem Zustand einen Teil der Werkzeukavität bilden und welche in offenem Zustand eine Einspritzöffnung bilden, deren Querschnitt etwa dem grössten Querschnitt der Werzeugkavität entspricht.

12. Vorrichtung nach Anspruch 9, **dadurch gekennzeichnet, dass** das Werkzeug eine in die Werkzeugkavität einschiebbare Einspritznadel (25) zum Einspritzen der Kernkomponente aufweist.

## Claims

1. A method of producing a multi-layer, physiologically compatible administration form, in particular a capsule, in which at least one core component is completely enclosed by at least one casing component, **characterised by** injection of the core component (2) and the casing component (3) into a common tool cavity (7), wherein at least the casing component is thermoplastically processed.

2. A method according to claim 1 **characterised in that**
- the core component is provided in a first feed device (4),
- the casing component is provided in the thermoplastic condition in a second feed device (5),
- the core component and the casing component are injected simultaneously or sequentially into the tool cavity in such a way that at the latest there the core component is completely surrounded by the casing component,
- and the injected moulding (1) is cooled and ejected after opening of the tool cavity (7).

3. A method according to claim 2 **characterised in that** the core component and the casing component are injected into the tool cavity (7) in the form of separate flows of molten material by way of a common injection head (10).

4. A method according to claim 2 **characterised in that** the core component and the casing component are injected into the tool cavity in the form of a common flow of molten material by way of a common injection head.

5. A method according to claim 4 **characterised in that** the core component is already enclosed by the casing component in the injection head, the common flow of molten material being injected into the opened mould cavity.

6. A method according to claim 2 **characterised in that** the tool cavity is firstly completely filled with the casing component by way of an injection opening and then the core component is injected into the casing component by way of an injection needle, wherein the casing component which is displaced **in that** situation flows out of the tool cavity by way of the injection opening.

7. A method according to one of claims 1 to 6 **characterised in that** at least the casing component is thermoplastically prepared in an extruder and the injection pressure is produced at the extruder.

8. A method according to one of claims 1 to 7 **characterised in that** the core component is supplied in metered form by means of a metering piston pump.

9. Apparatus for producing a multi-layer, physiologically compatible administration form, namely a capsule, comprising at least one core component which is completely enclosed by at least one casing component, for carrying out the method according to one of claims 1 to 8, **characterised by**
- a first feed device (4) in the form of an extruder for the casing component,
- a second feed device in the form of an extruder (5) or a metering piston pump (24) for the core component,
- a tool (6) comprising a tool block (13) and an injection plate (8) or a slider plate (14) which together form at least one common tool cavity (7) for receiving both components,
- a tool actuation means for opening and closing the tool, and
- an ejector (9) mounted in the tool block for ejecting the finished capsule from the tool cavity, wherein the tool cavity (7) is in the shape of a round, oval, oblong or ampoule-shaped capsule and wherein the separation plane between the tool block and the injection plate or slider plate is so selected that it extends through the tool cavity and that the capsule remains in the tool block upon opening of the tool and can be ejected by the ejector towards the separation plane.

10. Apparatus according to claim 9 **characterised in that** the core component can be meteredly fed with the metering piston pump either into the tool cavity or into the feed passage of the first feed device.

11. Apparatus according to claim 9 **characterised in that** the tool has a slider plate with at least two sliders (15, 15') which in the closed condition form a part of the tool cavity and which in the open condition form an injection opening, the cross-section of which approximately corresponds to the largest cross-section of the tool cavity.

12. Apparatus according to claim 9 **characterised in that** the tool has an injection needle (25) which can be inserted into the tool cavity for injection of the core component.

## Revendications

1. Procédé pour fabriquer une forme de présentation multicouche physiologiquement tolérable, en particulier une capsule, selon lequel au moins un composant intérieur est complètement enrobé par au moins un composant d'enrobage, **caractérisé par** l'injection du composant intérieur (2) et du composant d'enrobage (3) dans une cavité d'outil commun (7), le composant d'enrobage, au moins, subissant un traitement thermoplastique.

2. Procédé selon la revendication 1, **caractérisé**
- **en ce que** le composant intérieur est préparé dans un premier dispositif d'amenée (4),
- **en ce que** le composant d'enrobage est amené à l'état thermoplastique dans un second dispositif d'amenée (5),
- **en ce que** le composant intérieur et le composant d'enrobage sont injectés simultanément ou successivement dans la cavité d'outil de telle sorte que là, au plus tard, le composant intérieur soit complètement entouré par le composant d'enrobage,
- et **en ce que** le produit moulé par injection (1) est refroidi et est éjecté après l'ouverture de la cavité d'outil (7).

3. Procédé selon la revendication 2, **caractérisé en ce que** le composant intérieur et le composant d'enrobage sont injectés dans la cavité de moule (7) sous la forme de courants de masse fondue séparés, par l'intermédiaire d'une tête d'injection commune (10).

4. Procédé selon la revendication 2, **caractérisé en ce que** le composant intérieur et le composant d'enrobage sont injectés dans la cavité d'outil sous la forme d'un courant de masse fondue commun, par l'intermédiaire d'une tête d'injection commune.

5. Procédé selon la revendication 4, **caractérisé en ce que** l'enrobage du composant intérieur avec le composant d'enrobage a lieu dès la tête d'injection, le courant de masse fondue commun étant injecté dans la cavité d'outil ouverte.

6. Procédé selon la revendication 2, **caractérisé en ce que** la cavité d'outil est tout d'abord complètement remplie avec le composant d'enrobage par une ouverture d'injection, et **en ce que** le composant intérieur est ensuite injecté dans le composant d'enrobage par l'intermédiaire d'un pointeau d'injection, le composant d'enrobage qui est alors refoulé sortant alors de la cavité d'outil par l'ouverture d'injection.

7. Procédé selon l'une des revendications 1 à 6, **caractérisé en ce que** le composant d'enrobage, au moins, subit un traitement thermoplastique dans une extrudeuse et **en ce que** la pression d'injection est produite au niveau de l'extrudeuse.

8. Procédé selon l'une des revendications 1 à 7, **caractérisé en ce que** le composant intérieur est ajouté de manière dosée à l'aide d'une pompe de dosage à piston.

9. Dispositif pour fabriquer une forme de présentation multicouche physiologiquement tolérable, à savoir une capsule, comportant au moins un composant intérieur qui est complètement enrobé par au moins un composant d'enrobage, pour la mise en oeuvre du procédé selon l'une des revendications 1 à 8, **caractérisé par**
- un premier dispositif d'amenée (4) qui a la forme d'une extrudeuse pour le composant d'enrobage,
- un second dispositif d'amenée qui a la forme d'une extrudeuse (5) ou d'une pompe de dosage à piston (24) pour le composant intérieur,
- un outil (6) formé d'un bloc d'outil (13) et d'une plaque d'injection (8) ou d'une plaque à coulisseaux (14) qui définissent ensemble au moins une cavité d'outil commune (7) pour recevoir les deux composants,
- un actionnement d'outil pour ouvrir et fermer l'outil,
- et un éjecteur (9) qui est monté dans le bloc d'outil pour éjecter de la cavité d'outil la capsule finie, la cavité d'outil (7) ayant la forme d'une capsule ronde, ovale, oblongue ou en forme d'ampoule et le plan de séparation entre le bloc d'outil et la plaque d'injection ou la plaque à coulisseaux étant choisi de telle sorte qu'il traverse la cavité d'outil et que la capsule reste dans le bloc d'outil, lors de l'ouverture de l'outil, et puisse être éjecté par l'éjecteur en direction du plan de séparation.

10. Dispositif selon la revendication 9, **caractérisé en ce que** le composant intérieur est apte à être ajouté de manière dosée à l'aide de la pompe de dosage à piston soit dans la cavité d'outil, soit dans le conduit d'amenée du premier dispositif d'amenée.

11. Dispositif selon la revendication 9, **caractérisé en ce que** l'outil comporte une plaque à coulisseaux avec au moins deux coulisseaux (15, 15') qui, en position fermée, définissent une partie de la cavité d'outil et qui, en position ouverte, définissent une ouverture d'injection dont la section transversale correspond à peu près à la section transversale maximale de la cavité d'outil.

12. Dispositif selon la revendication 9, **caractérisé en ce que** l'outil comporte un pointeau d'injection (25) apte à être glissé dans la cavité d'outil pour injecter le composant intérieur.
